# EUROPEAN PATENT SPECIFICATION

(11) **EP 2 229 940 B1**
(45) Date of publication and mention of the grant of the patent: **01.07.2015**
(21) Application number: 10157239.4
(22) Date of filing: 22.03.2010
(51) Int. Cl.: A61K 31/12, A61K 31/352, A61K 45/06, A61K 9/48

(54) **Pharmaceutical composition presenting anti-inflammatory and anti-histaminic properties**
Pharmazeutische Zusammensetzung mit entzündungshemmenden und antihistaminischen Eigenschaften
Composition pharmaceutique présentant des propriétés anti-inflammatoires et antihistaminiques

(30) Priority: 31.12.2009 EP 09181042; 20.03.2009 EP 09155803
(43) Date of publication of application: 22.09.2010
(73) Proprietor: Bioxtract S.A., 1348 Louvain-la-Neuve (BE)
(72) Inventor: Priem, Fabian, B-1332, Genval (BE); Jacquemond-Collet, Ingrid, B-5001, Belgrade (BE)
(74) Representative: Pronovem

(56) References cited:
- WO-A2-2006/130679
- US-A- 6 056 971
- US-B1- 6 352 712
- CAI T ET AL: "Serenoa repens associated with Urtica dioica (ProstaMEV<(>R)) and curcumin and quercitin (FlogMEV<(>R)) extracts are able to improve the efficacy of prulifloxacin in bacterial prostatitis patients: results from a prospective randomised study" INTERNATIONAL JOURNAL OF ANTIMICROBIAL AGENTS, ELSEVIER SCIENCE, AMSTERDAM, NL LNKD- DOI:10.1016/J.IJANTIMICAG.2008.11.012, vol. 33, no. 6, 31 January 2009 (2009-01-31), pages 549-553, XP002586296 ISSN: 0924-8579 [retrieved on 2009-01-31]
- AZIZAH ABDUL-HAMID ET AL: "Functional properties of dietary fibre prepared from defatted rice bran", FOOD CHEMISTRY, ELSEVIER LTD, NL, vol. 68, no. 1, 1 January 2000 (2000-01-01), pages 15-19, XP002664741, ISSN: 0308-8146, DOI: 10.1016/S0308-8146(99)00145-4 [retrieved on 1999-10-15]

## Description

### Field of the invention

The present invention relates to a new pharmaceutical, a nutraceutical (or a food (or a feed) additive) or a functional food (or feed) composition presenting an improved formulation of plant extracts to increase the bio-availability of the active components present in this composition, and its use in the treatment and/or the prevention of inflammation and an inflammation-related syndromes or disorders. as defined by the claims.

### State of the Art

Numerous agents identified from fruits and vegetables can interfere with several cell-signaling pathways. The agents include curcumin (turmeric), but also resveratrol (present in red grapes, peanuts and berries), genistein (present in soybean), diallyl sulfide (present allium), S-allyl cysteine (present in allium), allicin (present in garlic), lycopene (present in tomato), capsaicin (present in red chilli), diosgenin (present in fenugreek), 6-gingerol (present in ginger), ellagic acid (present in pomegranate), ursolic acid (present in apple, pears, prunes), silibinin (present in milk thistle), anethol (present in anise, camphor, and fennel), catechins (present in green tea), eugenol (present in cloves), indole-3-carbinol (present in cruciferous vegetables), limonene (present in citrus fruits), beta carotene (present in carrots), and dietary fibers (present in many vegetables and fruits).

US 6 352 712 B1 discloses dietary supplement comprising curcumin or quercetin, which are designed to act as medical food.

Cai et al., international Journal of Antimicrobial Agents, 2009, 549-553 disclose the combined use of extracts of Serenoa repens, Urtica dioica, quercetin and curcumin in addition to prulifloxacin for use in patients affected by chronic bacterial prostatitis.

The cell-signaling pathways inhibited by curcumin seems to include one or more of the following biological factors NF-κB, AP-1, STAT3, Akt, Bcl-2, Bcl-XL, caspases, PARP, IKK, EGFR, HER2, JNK, MAPK, COX2, and 5-LOX.

Curcumin is the principal curcuminoid of the popular Indian curry spice turmeric. The curcuminoids are polyphenols and are responsible for the yellow color of turmeric.

Extensive work has been done in attempts to establish the biological activities and pharmacological actions of curcumin, at least *in vitro* (including experiments carried-out on cultured cell). Curcumin may be beneficial for its antitumor (cancer), antioxidant, antiarthritic, anti-ischemic, anti-diabetic, anti-amyloid, and anti-inflammatory properties, in addition to possible benefits in cardiovascular diseases, neurological diseases and in Crohn's disease.

However, at neutral and basic pH, curcumin is rapidly degraded *in vitro.*

In addition, when eaten, little curcumin is absorbed: curcumin undergoes avid intestinal metabolism and its intestinal absorption is low, precluding reaching blood concentrations of curcurmin compatible with effects demonstrated *in vitro.*

It is postulated in the art that blood concentrations of curcumin (and its derivatives) of about 0.5 µM to about 6 µM are safe, and compatible with the advantageous effects measured *in vitro.*

Besides the metabolism, rapid systemic elimination of curcumin further contributes to the rather limited clinical effect.

To improve its bioavailability (at the plasma and tissue levels), numerous approaches have been undertaken. These approaches may involve the use of adjuvants, like piperine. Indeed, piperine interferes with glucuronidation, a physiological mechanism (occurring in the liver and small intestine) of modification of compounds foreign to an organism's normal biochemistry (such as drugs and poisons), in order to increase their water solubility and their excretion.

Co-supplementation with 20 mg of piperine (extracted from black pepper) may increase the plasma concentration of curcumin.

However, due to its effects on drug metabolism, (i.e., inhibition of glucuronidation) piperine should be taken cautiously (if at all) by individuals taking other medications.

Alternatively, dissolving curcumin in hot water prior to ingestion, or in warm oily liquids, appears to increase its bioavailability, but this fact is not well established and heat instability of curcumin is frequently postulated.

Heat- and light- stability of curcumin appear to be increased by its encapsulation into cyclodextrin (Szente et al., 1998, Journal of inclusion Phenomena and Molecular Recognition in Chemistry, 32, 81-89).

Other stable metabolites of curcumin, such as tetrahydrocurcumin are used in topical application and possibly found *in vivo,* but it is not clear if they provide the same therapeutic activity as curcumin.

The presence of acids increases the stability of curcumin, but decreases its solubility. Conversely basic environment increase the curcumin solubility, but at the expense of a sharply reduced stability.

Therefore, caution must be taken when formulating more bio-available curcumin.

Compositions containing curcumin and alginate or curcumin and gelatine have a solubility increased by >10⁴ fold at pH=5. However, curcumin was not stabilized towards hydrolytic or photolytic degradation.

The use of liposomal curcumin or curcumin nanoparticles or curcumin phospholipid complex may be beneficial.

For instance, nanoparticles comprising curcumin have a size of less than 100 nanometers on average demonstrated comparable to superior efficacy compared to free curcumin in human cancer cell line models. However, actual *in vivo* absorption has not been demonstrated with these nanoparticles.

WO2007/101551 describes a method to increase the bioavailability of curcumin that involves a simple procedure creating a complex with soy phospholipids, however the obtained plasma concentration of curcumin using this formulation only reached 0.033 micromolar.

US 6 056 971 B1 discloses a method for enhancing the dissolution properties of insoluble dietary supplements with an emulsifier. Coenzyme Q-10 when formulated with Span® 80, Tween® 80 and propylene glycol was more bio-available. Curcumin is disclosed in a mixture with Span® 80, Tween® 80 and propylene glycol before encapsulation but there is no mention of the bio-availability of this formulation.

WO2006/130679 describes compositions containing chemicals having functional carbonyl groups for the treatment of allergic rhinitis.

It is suggested in this international patent application WO2006/130679 that curcumin (that bears carbonyl groups) may possibly react *in vitro* with histamine through the formation of Schiff bases involving a carbonyl group of curcumin and the amine group of histamine, and it is therefore speculated that compounds having functional carbonyl groups might be of value for a treatment of allergic rhinitis (*in vivo*).

In one example of the patent application WO2006/130679, a commercial mixture of curcumin and polysorbate is used.

However, in the patent application WO2006/130679, the commercial composition of curcumin with polysorbate was demonstrated to be ineffective in reacting with histamine.

Furthermore, this prior art teaches that too acid mixtures should be avoided, since no base Schiff can be formed at acidic pH.

Finally, no *in vivo* data are disclosed in the patent application WO2006/130679.

New methods resulting in enhanced *in vivo* effect of curcumin are therefore required to bring this promising natural product to the forefront of therapeutic agents for treatment of various human diseases.

Quercetin is a flavonoid known for significant anti-inflammatory activity by inhibiting both synthesis and release of histamine and other allergic/inflammatory mediators. In addition, it exerts potent antioxidant activity. Quercetin also shows antitumor properties *in vitro.*

Quercetin solubility is an issue for its absorption. Therefore, methods to increase its solubility present interest.

### Summary of the invention

The present invention as defined by the claims provides a new composition and methods to obtain and use this composition that does not present the drawbacks of the products of the state of the art, especially a composition and methods that improves the bioavailability of curcumin and of quercetin in mammal blood, especially in human blood.

Furthermore, the invention provides a composition that comprises only food ingredients or elements that are compatible for an administration as a food (or feed) additive or in a functional food (or feed) composition.

Finally, the invention provides such composition that can be used for improving the treatment and/or the prevention of inflammation or inflammation-related syndromes or disorders, while possibly requiring lower administration doses than the known composition of the state of the art.

The inventors have found a method to increase the bio-availability of active compound being curcumin and quercetin, for an animal (preferably a mammal, including a human) by providing these curcumin and quercetin in a composition containing an edible (and advantageously food compatible) emulsifier.

The present invention is related to a composition comprising in one capsule, an acid, a sufficient amount of curcumin, a sufficient amount of quercetin and a sufficient amount of emulsifier, wherein the sufficient amount of curcumin is comprised between 2 mg and 200 mg, the sufficient amount of quercetin is comprised between 10 mg and 200 mg and the sufficient amount to the emulsifier is comprised between 200 mg and 700 mg, the acid is comprised between 1 mg and 5 mg, wherein the emulsifier has a viscosity at 25°C comprised 0.100 Pa.s and 0.800 Pa.s, for use in the treatment and/or the prevention of chronic rhinitis and/or allergic rhinitis.

The composition according to the invention is encapsulated in gelatine capsules.

Preferably, in the composition according to the invention, the acid is an organic acid selected from the group consisting of citric acid (E330), malic acid (E296), acetic acid (E260), tartaric acid (E334), lactic acid (E270), alginic acid (E400) and a mixture thereof.

Preferably, in the composition according to the invention, the sufficient amount of the curcurmin is comprised between 10 mg and 100 mg and between 5 mg and 50 and wherein the sufficient amount of quercetin is comprised between 20 mg and 100 mg.

The composition according to the invention further comprises a dryer, preferably selected from the group consisting of cellulose, cellulose derivatives and SiO₂.

Preferably, the dryer is SiO₂ having a mean particle size is comprised between 5 µm and 100 µm.

According to a preferred embodiment of the present invention, the composition according to the invention further comprises a sufficient amount of an essential oil which is comprised between 3 % (w:w) to 30 % (w:w) of essential oil, which is selected from the group consisting of Agar oil, Ajwain oil, Anise oil, Balsam oil, Parsley oil, Bergamot oil, Cardamom seed oil, Carrot seed oil, Chamomile oil, Calamus oil, Cinnamon oil, Citronella oil, Fennel seed oil, Fenugreek oil, Frankincense oil, Galangal oil, Geranium oil, Ginger oil, Grapefruit oil, Henna oil, Juniper berry oil, Lavender oil, Lemongrass oil, Melissa oil, Mint oil, Myrrh oil, Orange oil, Oregano oil, Orris oil, Peppermint oil, Pine oil, Rosehip oil, Sage oil, Schisandra oil, Spikenard, Tarrangon oil, Tea tree oil, Thyme oil, Turmeric oil, Valerian oil, Yarrow oil and Zedoary oil.

In the composition according to the invention, the essential oil is Turmeric oil.

By inflammation-related syndrome or disorder, it is meant a mammal (human) disease caused by or presenting an inflamed status.

More precisely, inflammation-related syndromes or disorders encompass cancer, cardiovascular diseases, preferably selected from the group consisting of atherosclerosis and ischemic heart (ischemia) disease, asthma, auto-immune diseases, chronic inflammation, chronic prostatitis, glomerulonephritis, hyper sensitivities, inflammatory bowel disease, pelvic inflammatory disease, reperfusion injury, auto-immune diseases or allergic disease, such as rhinitis, osteoarthritis (rheumatoid arthritis), transplant rejection, vasculitis, abdominal obesity or neurodegenerative diseases, such as Alzheimer disease, Parkinson disease or Huntington disease.

These syndromes or disorders include also allergic reactions formerly known as type 1 hyper sensitivity (that are the result of an inappropriate immune response (inflammation-related disorder) triggering inflammation) and other hyper sensitivity reactions (type 2 and type 3) mediated by antibody reactions and that induces inflammation by attracting leukocytes, which damage surrounding tissues. These syndromes and disorders include also inflammatory myopathies caused by the immune system inappropriately attacking component of muscle leading to muscle inflammation (that may occur in conjunction with other immune disorders, such as systemic sclerosis and including dermatomyositis, polymyositis and inclusion body myositis). These syndromes and disorders include also high level of several inflammations relating markers, preferably selected from the group consisting of IL-6, IL-8 and TNF-alpha that are associated with obesity.

Prolonged inflammation (inflammation-related disorder), known as acute or chronic inflammation leads to a progressive shift of the type of cells which are present at the site of inflammation and is characterized by simultaneous destruction and healing of the tissue from the inflammatory process that could lead to these known chronic diseases syndromes or disorders, preferably selected from the group consisting of diabetes mellitus, cardio- and cerebrovascular diseases like hypertension or ischemia, auto-immune diseases (including diseases of inflammatory origin like arthritis or lupus), brain diseases (including neuro-degenerative diseases, like Alzheimer disease, Parkinson disease, Huntington disease, multiple sclerosis, depression or schizophrenia), asthma, systemic sclerosis, allergies and cancer, one or all the above identified disease being treated and/or prevented by the composition of the invention.

Osteoarthritis is a preferred disease to be treated and/or prevented with the composition of the invention.

Chronic rhinitis and/or allergic rhinitis are the most preferred disease(s) treated and/or prevented with the composition of the invention.

Another consequence of inflammation-related disorders is pain that is also to be treated and/or prevented by the composition of the invention.

Diseases involving pain in connective tissues, such as fibromyalgia, are also a syndrome or a disorder to be treated and/or prevented of the present invention.

Therefore, in the present description, the applicant will use for the same effect, the words "chronic inflammation", "chronic inflammation associated disease" or "inflammatory disorders" that constitute a large and related group of syndromes and disorders collectively named inflammation-related syndrome or disorder, which underlies a variety of mammal (human) diseases.

Advantageously, the pharmaceutical composition of the present invention is combined with (encapsulated in) an adequate carrier, preferably a capsule, bead, or tablet), that is preferably food compatible, such as gelatine capsule, that is more preferably coated with an (food compatible) element that renders this composition resistant to gastric digestion in the stomach, more preferably this element being E904 (Shellac).

Advantageously, the composition (either in the form of a non aqueous solution or a solid composition) of the invention comprising a sufficient amount of curcumin and of quercetin, a sufficient amount of an emulsifier and possibly of a sufficient amount of an acid, is taken orally and is a per os (pharmaceutical or neutraceutical or functional food/feed) composition.

By 'about', it is preferably meant a variation of plus or minus 10%. For instance about 5 % means every number from 4.5% to 5.5%.

Advantageously, this composition is suitable to be taken orally, once, twice or thrice a day and/or advantageously 1, 2, 3, 4, 5, 6, 7, 8, 9 or up to 10 capsules, preferably 1 to 6 capsules, more preferably 2 to 4 capsules of the invention are taken by a subject on a daily basis.

The oral composition of the invention may further comprise a sufficient amount of an essential oil.

Preferred essential oils according to the invention are selected from the group consisting of Agar oil, Ajwain oil, Anise oil, Balsam oil, Parsley oil, Bergamot oil, Cardamom seed oil, Carrot seed oil, Chamomile oil, Calamus oil, Cinnamon oil, Citronella oil, Fennel seed oil, Fenugreek oil, Frankincense oil, Galangal oil, Geranium oil, Ginger oil, Grapefruit oil, Henna oil, Juniper berry oil, Lavender oil, Lemongrass oil, Melissa oil, Mint oil, Myrrh oil, Orange oil, Oregano oil, Orris oil, Peppermint oil, Pine oil, Rosehip oil, Sage oil, Schisandra oil, Spikenard, Tarrangon oil, Tea tree oil, Thyme oil, Turmeric oil, Valerian oil, Yarrow oil and Zedoary oil, being more preferably Turmeric oil.

Advantageously, in the composition of the invention, from about 3% (w:w) to about 30% (w:w), preferably from about 5% (w:w) to about 15% (w:w), more preferably about 7% (w:w) of essential oils is present (added (to the others elements of the composition of the invention).

In the pharmaceutical (or nutraceutical) composition of the invention, preferably from about 25 ml to about 200 ml, more preferably about 50 ml of (water-free) essential oils is added.

By essential oils, it is meant volatile and liquid (but non-aqueous) aroma compounds from natural sources, usually plants, with a poor solubility in water.

Essential oils are usually prepared by extraction techniques, preferably selected from the group consisting of such as distillation (including steam distillation), cold pressing, extraction (maceration) and/or solvent extraction.

The essential oils of the invention preferably essentially consist of terpen(-related) compounds such as monoterpens and/or sesquiterpens and are preferably water-free.

By essentially consisting of terpen (-related) compounds, it is meant that at least 50% (on a molar basis), preferably at least 70%, more preferably at least 80% of the essential oil or all the essential oils are made of terpen(-related) compounds and are preferably water-free.

Terpens are derived biosynthetically from units of isoprene. Monoterpens consist of two isoprene units and sesquiterpens consist of three isoprene units. To the isoprene (monoterpens and/or sesquiterpens) backbone, additional hydrolysis and/or rearrangements and oxidations provide the final terpens compound of an essential oil.

Alternatively, the most abundant molecules of an essential oil (terpens compounds from essential oils) may be used in the composition of the invention, instead of the complex essential oil, for instance terpens (-related) compounds, preferably selected from the group consisting of ar-Turmerone, Turmerone, Curlone, ar-Curcumene and p-Cymene and a mixture thereof.

Advantageously, several emulsifiers are present and combined (mixed) in the composition of the invention, provided their mean HLB, viscosity, content and ratio of content is kept within the disclosed ranges.

Preferred emulsifiers according to one embodiment of the invention have a low viscosity and good water solubility.

Advantageously, the emulsifier(s) present in the composition according to the present invention has (have) a low viscosity (25°C) of about 0.100-0.800 Pa.s, combined to a medium (6-10) to high (11-25) hydrophilic-lipophilic balance (HLB), being preferably Polysorbate 80.

Advantageously, in the composition (in the form of a non aqueous solution) according to the invention, the sufficient amount of the low viscosity emulsifier(s) is comprised between about 200 mg and about 700 mg, more preferably between about 400 mg and about 700 mg, still more preferably between about 600 mg and about 700 mg, preferably between about 600 mg and about 650 mg.

Advantageously, in the composition (in the form of a non aqueous solution) according to the invention, the sufficient amount of the curcurmin is comprised between about 5 mg and about 200 mg, preferably between about 10 mg and about 100 mg, more preferably between about 30 mg and about 50 mg, more preferably about 40 mg (or more preferably of about 42 mg).

Advantageously, in the composition (in the form of a non aqueous solution) according to the invention, the sufficient amount of the quercetin is comprised between about 10 mg and about 200 mg, preferably between about 20 mg and about 100 mg, and is more preferably of about 75 mg (alternatively the preferred quercetin content is of about 65 mg).

Preferably, the ratio of curcumin to the low viscosity emulsifier(s) is comprised between about 1:99 and about 30:70 (w:w), more preferably between about 5:95 and about 15:85 (w:w), still more preferably between about 5:95 and about 12:88 (w:w), still more preferably of about 6:94 (w:w).

Preferably, the ratio of quercetin to the low viscosity emulsifier(s) is comprised between about 2:98 and about 30:70 (w:w), more preferably between about 10:90 and about 20:80 (w:w), still more preferably of about 10:90 (w:w) (alternatively, the preferred ratio is of about 9:91; w:w).

Advantageously in the (non aqueous solution) composition according to the invention, the acid component is present at a concentration comprised between about 0.5 mg and about 35 mg, more preferably between about 1 mg and about 10 mg, more preferably between about 2 mg and about 5 mg, more preferably about 3 mg (alternatively, the preferred concentration is of about 3.5 mg).

Possibly, the (non aqueous solution) composition according to the invention further comprises a sufficient amount of an (or a mixture of) essential oil(s) (monoterpens and/or sesquiterpens).

Alternatively, in another embodiment of the invention, the emulsifier(s) present in the (solid) composition according to the present invention has (have) a high viscosity (50°C) comprised between about 75.0 and about 175.0 Pa.s, combined to a medium (6-10) to high (11-25) hydrophilic-lipophilic balance (HLB), this emusifier being preferably Polysorbate 60.

Advantageously, in the (solid) composition according to the invention, the sufficient amount of the high viscosity emulsifier(s) is comprised between about 100 mg and about 700 mg, more preferably between about 200 mg and about 500 mg, still more preferably between about 300 mg and about 500 mg.

Advantageously, in the (solid) composition according to the invention, the sufficient amount of the curcurmin is comprised between about 2 mg and about 100 mg, preferably between about 5 mg and about 50 mg, more preferably of between about 10 mg and about 30 mg, more preferably about 16 mg.

Advantageously, in the (solid) composition according to the invention, the sufficient amount of the quercetin is comprised between about 10 mg and about 200 mg, preferably between about 20 mg and about 100 mg, more preferably about 25 mg.

Preferably, the ratio of quercetin to the high viscosity emulsifier(s) is comprised between about 2:98 and about 30:70 (w:w), more preferably between about 10:90 and about 20:80 (w:w)), still more preferably of about 10:90 (w:w). Alternatively, the preferred ratio is of about 9:91 (w:w).

Preferably the ratio of curcumin to the high viscosity emulsifier(s) present in the composition is comprised between about 1:99 and about 20:80 (w:w), more preferably between about 5:95 and about 15:85 (w:w), still more preferably between about 5:95 and about 12:88 (w:w), still more preferably of about 6:94 (w:w).

Advantageously, in the (solid) composition according to the invention, the acid component is present at a concentration comprised between about 0.25 mg and about 35 mg, more preferably between about 1 mg and about 10 mg, more preferably between about 1 mg and about 5 mg, more preferably about 1.5 mg.

Advantageously, the (solid) composition comprises sufficient amount of curcumin, of quercetin, of (a) high-viscosity emulsifier(s), of an acid and of a dryer.

Possibly, the (solid) composition according to the invention further comprises a sufficient amount of an (or a mixture of) essential oil(s) (monoterpens and/or sesquiterpens being preferably water-free).

The dryer present in the composition is any (inert and food compatible) substance able to adsorb (and therefore to provide a solid aspect like a powder) the high-viscosity emulsifier(s) and being preferably selected from the group consisting of cellulose, cellulose derivatives, such as methylcellulose, hydroxypropylcellulose (and combinations thereof), and SiO₂.

The preferred dryer according to the invention is SiO₂.

Advantageously, the amount of the dryer (preferably SiO₂ particules) is comprised in the composition between about 50 mg and about 400 mg, preferably between about 100 mg and about 300 mg, more preferably about 120 mg.

Preferably, this dryer (preferably SiO₂) is added to the pharmaceutical composition at 1:10 to about 5:10, more preferably at about 3:10 (w:w).

Preferably, the SiO₂ particules added to the composition of the invention are made of a silica of high absorptive capacity.

Preferably, the SiO₂ particules added to the composition of the invention is made of a silica of a low particle size, with an average diameter ranging from about 5 µm to about 100 µm, possibly sold under the name Sipernat 50S.

Preferably, the SiO₂ of the invention is non-derivatized.

Another aspect of the present invention is a method for the treatment of inflammatory disease by the step of administrating a sufficient amount of the composition of the invention to a mammal subject (preferably a human patient), possibly suffering from one of the above mentioned diseases, syndromes and disorders.

More preferably in the method of the invention, from about 20 mg to about 500 mg of curcumin (preferably from about 40 mg to about 120 mg of curcumin) and from about 20 mg to about 1000 mg of quercetin (preferably about 150 mg of quercetin or about 130 mg of quercetin) is administrated on a daily basis to this mammal subject (preferably to an adult human patient) to reach blood concentration of this curcumin and curcumin derivatives to a concentration value of at least (about) 0.02 µM (in an adult human), preferably of at least 0.05 µM, and a blood concentration of quercetin of at least (about) 0.5 µM, more preferably of at least (about) 0.7 µM.

A last aspect of the invention is a method to obtain the composition of the invention comprising (consisting of) the steps of:
- preparing or obtaining substantially pure curcumin and quercetin,
- dissolving the substantially pure curcumin in an adequate amount of emulsifier and advantageously an organic acid,
- optionally adding a sufficient amount of an (or a mixture of) essential oils (monoterpens and/or sesquiterpens),
- dissolving substantially pure quercetin in the composition comprising curcumin, an emulsifier and possibly an essential oil,
- optionally mixing the composition consisting of this curcumin, of this quercetin and of this emulsifier onto a dryer, and crushing particles,
- filling a (gelatine) capsule with this composition comprising (consisting of) this curcumin, this quercetin and this emulsifier and possibly this dryer into, and
- possibly obtaining an enteric coating (with addition of E904 layer(s)) of this (gelatine) capsule.

Preferably, the gelatine capsule (in the liquid form) allows to package from about 700 mg to about 1000 mg of the composition of the invention. Preferably, the gelatine capsule (in the solid form) allows to package of about 400 mg of the composition of the invention.

### Detailed description of the invention

### Definitions

Curcumin is also known as [1,7-bis(4-hydroxyl-3-methoxyphenyl)-1,6-heptadiene-3,5-dione, as diferuloylmethane or as E100 food additive.

The present invention encompasses curcumin, including its keto and enol forms, but preferably not the chemically-derived forms of curcumin nor di-tetra- or hexa-hydrocurcumin.

Quercetin is a plant-derived flavonoid that occurs naturally in apples, tea or onions. Quercetin may be found in glycosylated forms, resulting into an increased bio-availability.

Advantageously, quercetin used in the invention is not glycosylated.

Polysorbate 80, E433, Tween®80 or PEG 20 Sorbitan monooleate is an emulsifier.

Polysorbate 60, E435, Tween®60, PEG 20 Sorbitan monostearate is an emulsifier.

By viscosity, it is meant dynamic viscosity, expressed in Pa*s.

By emulsifier, it is meant edible compounds that are amphiphilic and therefore have the potential to reduce the interfacial tension between an hydrophobic compound (such as curcumin, quercetin and essential oil) and water by adsorbing at the liquid-liquid interface. The emulsifiers of the present invention may assemble into aggregates, such as micelles that can encapsulate the hydrophobic compound of the invention.

Surprisingly, the inventors found that acid mixtures of curcumin and quercetin with the emulsifiers and possibly with essential oils (monoterpens and/or sesquiterpens) according to the invention result into improved stability of curcumin and into a sustained solubility of both compounds in duodenal conditions (e.g. in a phosphate buffer at pH 6.8, 37°C), as well as bio-availability.

### Examples

### Example 1 :

Commercial curcumin having a purity of at least 90% (the inventors preferably selected curcumin batches having a purity of at least 95% or 96% and possibly close to 100%) is used. These lots are obtained after extraction with ethanol and re-crystallization using methanol.

### Preparation of the liquid extract

The curcumin preparation is preferably made using curcumin powder with fine granulometry (having the preferred and advantageous granulometry comprised between about 100 µm and about 200 µm). Firstly, the citric acid crystals (having advantageously granulometry below 150 µm) are mixed with curcumin. The resulting mixture is stirred into Polysorbate 80. After heating to 30°C for adequate homogenisation, this complete mixture is mixed for 45 min. Then the whole mixture is milled with a three-roll-mill (preferably Coball mill) and closing aerated with nitrogen to remove present air. The preparation is then encapsulated shortly in gelatine capsules, preferably an enteric coated capsules with addition of E 904 (SHELLAC), preferably about 700 mg per capsule.
The concentration of pure curcumin is preferably 6%, with 0.5% citric acid completed to 100% with Polysorbate 80.

### Example 2:

The preparation is made as in example 1, but using a high viscosity emulsifier, preferably Polysorbate 60. SiO₂ is then added to a final percentage of 10% to 60%, preferably 30% to 40% max (more preferably about 30%) in a planetary mixer or in a high sheer blender, until obtaining a homogenous and fluid powder. This powder can be compressed into a tablet or filled into hard shell capsules (preferably 400 mg per capsule).

The concentration of pure curcumin in the final composition is preferably of 4%, with 0.35% citric acid and a final concentration of SiO₂ preferably of 30%. All percentages are on a weight by weight (w:w) basis.

### Example 3 :

Solubility of curcumin in the liquid composition of Example 1 is measured by agitating 1.4 g (equivalent to the posology of 2 capsules of 700 mg per day) of this preparation (containing 84 mg of curcumin) in 900 ml of phosphate buffer pH 6.8, for 1 hour at 37°C.

In comparison, the same amount of curcumin (84 mg) is solubilised in phosphate buffer pH 6.8, for 1 hour at 37°C.

After 1 hour, the solution is filtered on a 0.2 µm filter and the solubilized curcumin is quantified. Solubilized curcumin in the liquid composition of Example 1: 35% of the initial dose.

Curcumin in composition of Example 1 is 8750 times more soluble than curcumin without polysorbate and citric acid.
Solubility of curcumin in the powder composition of example 2 is measured by agitating 1.2 g (equivalent to the posology of 3 capsules of 400 mg per day) of this preparation (containing 48 mg of curcumin) in 900 ml of phosphate buffer pH 6.8, for 1 hour at 37°C.
In comparison, the same amount of curcumin (48 mg) is solubilised in phosphate buffer pH 6.8, for 1 hour at 37°C. After 1 hour, the solution is filtered on a 0.2 µm filter and the solubilized curcumin is quantified.

Solubilized curcumin in composition of Example 2: 30% of the initial dose.
Curcumin in Composition of Example 2 is 6000 times more soluble than curcumin without polysorbate and citric acid.

### Example 4 :

### Composition:

Curcumin preparation is made as in example 1 using curcumin powder with fine granulometry (100-200 µm). Firstly, the citric acid crystals (having a preferred and advantageous granulometry below 150 µm) are mixed with curcumin. The resulting mixture is stirred into Polysorbate 80 and Turmeric Essential Oil, extracted from rhizomes of *Curcuma longa* L. by supercritical extraction. It contains min. 60% of total turmerones (Aromatic-turmerones, alpha-turmerone and beta-turmerone). Hydrodistillation or steam distillation may be also applied to extract said essential oil. After heating to 30°C, the complete mixture is mixed for 45 min. Then the whole mixture is milled with a Coball Mill (three-roll-mill preferably) and closing aerated with nitrogen. The preparation is then encapsulated shortly in gelatine capsules, preferably enteric coated, preferably 700 mg per capsule.

The resulting composition is made of 6% (w:w) curcumin, 0.5% (w:w) citric acid and 7.14% (w:w) turmeric essential oil completed to 100% with Polysorbate 80 (86.36%; w:w).

### Solubility:

Solubility of curcumin in the liquid composition of example 4 is measured by agitating 1.4 g (equivalent to the posology of 2 capsules of 700 mg per day) of this preparation (containing 84 mg of curcumin) in 900 ml of phosphate buffer pH 6.8, for 1h at 37°C.

In comparison, the same amount of the curcumin preparation (84 mg) of Example 1 is solubilised in phosphate buffer pH 6.8, for 1h at 37°C.

After 1h, the solution is filtered on a 0.2 µm filter and the solubilized curcumin is quantified. Solubilized curcumin from Example 1: 35% of the initial dose.
Solubilized curcumin from Example 4 (with the addition of essential oil): 50% of the initial dose.

The inventors repeated the same experiment but with the replacement of turmeric essential oils by the same amount of Peppermint essential oil and obtained the same improved solubility over the curcumin composition comprising only an emulsifier.

As controls, the inventors mixed curcumin with essential oils and measured only a poor solubility of curcumin, close to the solubility of pure curcumin.

The inventors conclude that curcumin in a composition comprising an emulsifier and supplemented with essential oil is 12.500 times more soluble than a curcumin composition with essential oils but without emulsifier, and 1.5 more soluble than a curcumin composition with emulsifier and citric acid (without essential oils), although the amount of emulsifier is reduced in the curcumin composition with citric acid, emulsifier and essential oils.

### Example 5 : a composition comprising curcumin and quercetin

### Composition:

The curcumin preparation is preferably made using curcumin powder with fine granulometry (100-200 µm). Firstly, the citric acid crystals (having a preferred and advantageous granulometry below 150 µm) are mixed with curcumin. It is then mixed with curcumin. The resulting mixture is stirred into Polysorbate 80.

After heating to 30°C, quercetin extract (at least 95% pure (aglycone) quercetin) is added and the complete mixture is mixed for 45 min. Then the whole mixture is milled with a Coball Mill (three-roll-mill preferably) and closing aerated with nitrogen. The preparation is then encapsulated shortly in gelatin capsules, enteric coated or not, preferably about 768 mg per capsule (if quercetin extract is 95% quercetin pure).

### Solubility:

Solubility of curcumin and quercetin in the liquid composition of the above example is measured by agitating 1.54 g (equivalent to the posology of 2 capsules of 768 mg per day) of this preparation (containing 84 mg of pure curcumin and 130 mg of pure quercetin) in 900 ml of phosphate buffer pH 6.8, for 1 h at 37°C.
In comparison, the same amount of curcumin (84 mg) is solubilised in phosphate buffer pH 6.8, for 1 h at 37°C.

After 1 h, the solution is filtered on a 0.2 µm filter and the solubilized curcumin is quantified. Following the same protocol, the same amount of quercetin (150 mg) is solubilized in phosphate buffer pH 6.8.
Solubilized curcumin: 18% (16-20%) of the initial dose. Solubilized quercetin: 35% (30-40%) of the initial dose.

Solubility of curcumin without additives: 0.004% of the initial dose.
Solubility of quercetin without additives: 4% of the initial dose.
Curcumin in Composition 1 is 4.500 times more soluble than curcumin without polysorbate and citric acid. Quercetin in Composition 1 is 8.75 times more soluble than quercetin without polysorbate and citric acid.

The inventors conclude that water solubility of quercetin is strongly increased by the same emulsifier as for curcumin, at the expense of only a limited reduction in curcumin solubility.

### Example 6 :

### Oral Bioavailability Test of the composition comprising curcumin

The inventors firstly evaluated the acute toxicity of the formulations of the invention.

A pre-defined high dose (5000 mg/kg) of the formulation of Example 1 was given in a single oral dose to rats. Animals were daily observed for at least 14 days. The inventors noticed no toxicity.

Formulation of Example 1 was then studied in human volunteers to evaluate the relative oral bioavailability. Twenty-four healthy individuals volunteers aged between 18 and 55 years of age were selected for the study. The following treatments were administered as single doses: 1 "enteric-coated" capsule (6 males and 6 females) and 2 "enteric-coated" capsules (6 males and 6 females). "Enteric-coated" capsule is a gelatine capsule, coated with Shellac, and filled with 700 mg of a mixture of curcumin (6%; 42 mg), citric acid (0.5%; 3.5 mg) and as emulsifier polysorbate 80. Blood samples were taken at zero hour and periodically at one-hour or half-hour intervals for 12 hours (0, 0.5, 1, 1.5, 2, 3, 4, 6, 8 and 12 hours after dosing). At each sample collection, 6 ml of blood were sampled in heparin tubes. After centrifugation, plasma was transferred into two labelled tubes. The first tube was spiked with internal standard (clenbuterol) and extracted with methanol and acetonitrile. The extract was analysed by UPLC-MS/MS on a BEH-C18 column (100 x 2.1 mm, particles size 1.7 µm) using water + formic acid and acetonitrile + formic acid as solvent. The eluant flow rate was 0.6 ml/min and detection was achieved using an electrospray ionisation source (ESI) operating in positive ion mode. Efficiency of the extraction procedure for recovering curcumin from blood samples was determined by recovery of curcumin upon normal blood samples. The curcumin recovery was calculated at three different concentration levels with at least 5 replicates by comparing peak area for curcumin with those obtained by direct injection of reference solutions in triplicates at the same concentration levels.

No quantifiable trace of non-metabolized curcumin was observed irrespective of the subject, the time point and the dose.

The blood samples were then analysed after hydrolysis of potential conjugated metabolites of curcumin by means of β-glucuronidase and arylsulfatase. Samples were assayed for curcumin and its conjugates after incubating the plasma samples with the enzymes β-glucuronidase and sulfatase. For these assays, plasma samples (200 µL) were mixed with 20 µL of 1 M acetate buffer (pH 5.5), 5 µL of β-glucuronidase and arylsulfatase solution, and 20 µL of internal standard solution (clenbuterol; 2 µg/ml). The samples were incubated at 37°C for 3 h (determined after a test run). The samples were extracted with 1 mL of ethyl acetate/methanol (95:5; v/v). After centrifugation, the supernatant was evaporated gently to dryness under nitrogen stream at ambient temperature and reconstituted in 100 µL of methanol and 50 µL of water and 7.5 µL were injected into the UPLC system.

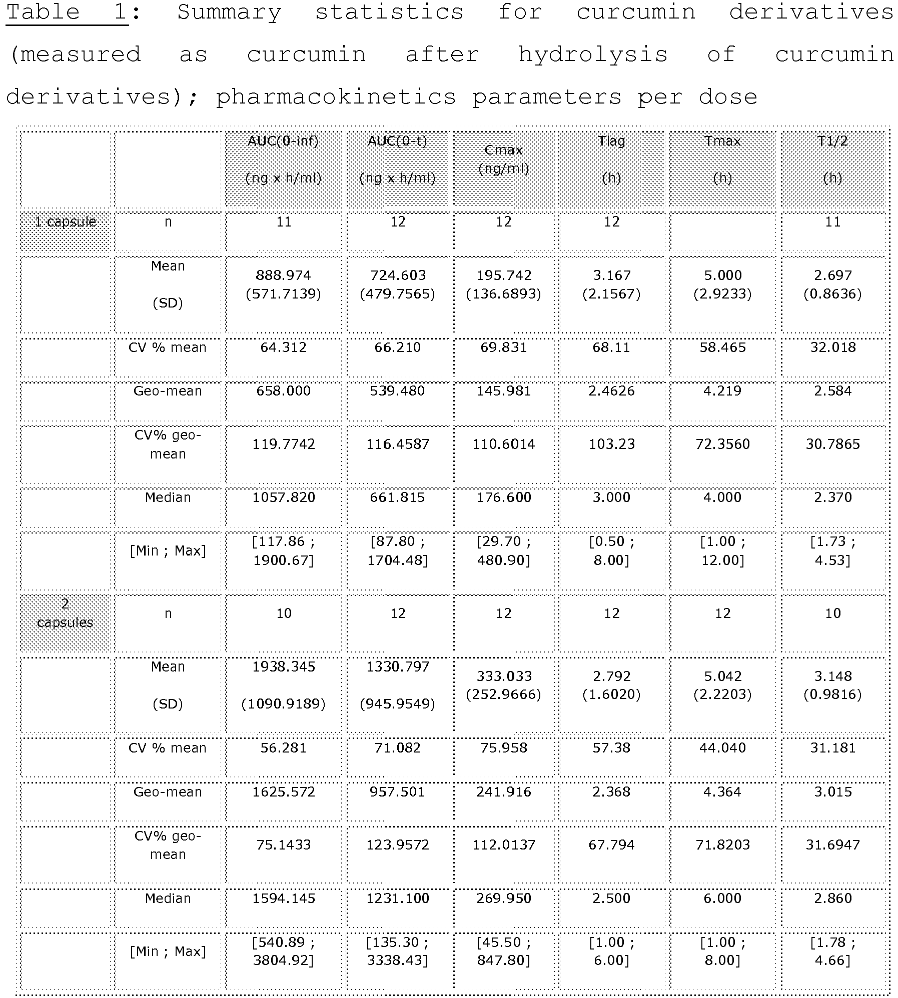

Different papers have demonstrated that such low dosages of oral curcumin (42 mg or 84 mg of curcumin) were not bioavailable at all, even under metabolised forms (glucuronides or sulfates of curcumin).

The inventors further confirmed this aspect by giving the same amount of curcumin in a 700 mg capsule with no emulsifier. They did not measured curcumin in blood, even after treatment with β-glucuronidase and arylsulfatase, as above.

The inventors then compared enteric-coated capsules with uncoated capsules comprising the same amount of curcumin and emulsifier.

After treatment with β-glucuronidase and arylsulfatase as above, the inventors noticed similar maximal blood values with a Cmax value of curcumin in blood of about 300-350 ng/ml in both cases.

### Example 7 :

### Oral Bioavailability Test of the composition comprising curcumin and quercetin

The inventors firstly evaluated the acute toxicity of the formulation of Example 5.

A pre-defined high dose (5000 mg/kg) of the formulation of Example 5 was given in a single oral dose to rats. Animals were daily observed for at least 14 days. The inventors noticed no toxicity.

Formulation of Example 5 was then studied in human volunteers to evaluate the relative oral bioavailabilities. Twelve healthy individuals volunteers aged between 18 and 55 years of age were selected for the study. The following treatment was administered as single doses: 2 non-enteric-coated capsules (6 males and 6 females). Non-Enteric-coated capsule is a gelatine capsule, filled with 768 mg of a mixture of curcumin (5.5%; 42 mg); quercetin (∼8.5%; 65 mg), citric acid (0.4%; 3.5 mg) and as emulsifier polysorbate 80. Urine samples were collected periodically with the following timing: 0-4 hours, 4-8 hours, 8-12 hours). Blood samples were taken at zero hour and periodically at one-hour or half-hour intervals for 12 hours (0, 0.5, 1, 1.5, 2, 3, 4, 6, 8, 12 and 24 hours after dosing). At each blood sample collection, 6 ml of blood were sampled in heparin tubes. After centrifugation, plasma was transferred into two labelled tubes. The first plasma tube (as well as urine tube) was spiked with internal standard (clenbuterol) and extracted with methanol and acetonitrile. The extract was analysed by UPLC-MS/MS on a BEH-C18 column (100 x 2.1 mm, particles size 1.7 µm) using water + formic acid and acetonitrile + formic acid as solvent. The eluant flow rate was 0.6 ml/min and detection was achieved using an electrospray ionisation source (ESI) operating in positive ion mode. Efficiency of the extraction procedure for recovering curcumin from blood and urine samples was determined by recovery of curcumin upon normal blood and urine samples. The curcumin recovery was calculated at three different concentration levels with at least 5 replicates by comparing peak area for curcumin with those obtained by direct injection of reference solutions in triplicates at the same concentration levels.

No quantifiable trace of non-metabolized curcumin was observed irrespective of the subject and the time point.

Cmax values of non-metabolized quercetin in blood were of 0.447 µM.

The blood samples were then analysed after hydrolysis of potential conjugated metabolites of curcumin and of quercetin by means of β-glucuronidase and arylsulfatase. Samples were assayed for curcumin and quercetin conjugates after incubating the plasma samples with the enzymes β-glucuronidase and sulfatase. For these assays, plasma samples (200 µL) were mixed with 20 µL of 1 M acetate buffer (pH 5.5), 5 µL of β-glucuronidase and arylsulfatase solution, and 20 µL of internal standard solution (clenbuterol; 2 µg/ml). The samples were incubated at 37°C for 3 h (determined after a test run). The samples were extracted with 1 mL of ethyl acetate/methanol (95:5; v/v). After centrifugation, the supernatant was evaporated gently to dryness under nitrogen stream at ambient temperature and reconstituted in 100 µL of methanol and 50 µL of water and 7.5 µL were injected into the UPLC system.

After this treatment with β-glucuronidase and arylsulfatase, the inventors noticed Cmax (maximal blood concentration) of curcumin in blood of 34 ng/ml.

Cmax of quercetin after this enzymatic treatment was of 225 ng/ml (Table 2).

The urinary recovery of total quercetin and total curcumin after enzymatic treatment was respectively 2.89% and 0.084%.

Different papers have demonstrated that such low dosages of oral curcumin (84 mg of curcumin) were not bioavailable at all, even under metabolised forms (glucuronides or sulfates of curcumin).

The blood concentration of quercetin was also further increased of about 2-fold upon the formulation of the invention by comparison to the ingestion of the same amount of non-glycosylated and non-formulated quercetin.

**Table 2: Summary statistics for quercetin and curcumin derivatives (measured as curcumin and quercetin after hydrolysis of derivatives) in plasma; pharmacokinetics parameters**

| **PLASMA LEVELS** | | **AUC(0-inf) (ng.h/mL)** | **AUC(0-t) (ng.h/mL)** | **Cmax (ng/mL)** | **Tlag (h)** | **Tmax (h)** | **t1/2 (h)** |
|---|---|---|---|---|---|---|---|
| Total Curcumin (glucuronide + sulfate) | n | 11 | 12 | 12 | 12 | 12 | 11 |
| | Mean (SD) | 349.675 (218.521) | 349.680 (223.796) | 34.175 (24.880) | 0.54 (0.14) | 6.458 (6.071) | 6.468 (2.432) |
| | CV% mean | 62.493 | 64.000 | 72.800 | 26.65 | 94.001 | 37.596 |
| | Geo-mean | 301.82 | 276.87 | 27.73 | 0.53 | 5.69 | 6.41 |
| | Median | 282.73 | 285.20 | 27.5 | 0.5 | 6.00 | 6.17 |
| | [Min; Max] | [109.637; 897.127] | [77.325; 858.225] | [10.8 ; 86.2] | [0.5 ; 1] | [1 ; 24] | [3.441 ; 10.665] |
| Quercetin Aglycone | n | 12 | 12 | 12 | 12 | 12 | 12 |
| | Mean (SD) | 86.577 (77.149) | 77.632 (76.757) | 142.191 (156.656) | 0.542 (0.144) | 0.625 (0.326) | 0.724 (0.612) |
| | CV% mean | 89.110 | 98.874 | 110.173 | 26.647 | 52.107 | 84.458 |
| | Geo-mean | 56.33 | 54.24 | 82.02 | 0.530 | 0.58 | 0.50 |
| | Median | 78.254 | 77.009 | 98.234 | 0.500 | 0.500 | 0.608 |
| | [Min; Max] | [5.935 ; 293.386] | [5.369 ; 292.722] | [6.735-579.39] | [0.5 ; 1] | [1 ;1.5] | [0.076; 2.226] |
| Total Quercetin (Aglycone + glucuronide + sulfate) | n | 11 | 12 | 12 | 12 | 12 | 11 |
| | Mean (SD) | 3293.605 (2847.306) | 1625.173 (1002.830) | 225.392 (148.118) | 0.250 (0.261) | 3.292 (6.689) | 15.964 (13.101) |
| | CV% mean | 86.450 | 61.706 | 65.716 | 104.447 | 203.222 | 82.066 |
| | Geo-mean | 2017.190 | 1238.376 | 169.196 | ND | 1.361 | 12.556 |
| | Median | 2869.415 | 1517.088 | 201.050 | 0.250 | 1.000 | 10.145 |
| | [Min; Max] | [115.00 ; 8301.86] | [106.875 ; 3551.650] | [13.600; 554.600] | [0; 0.5] | [1 ; 24] | [7.04 ; 50.12] |

**Table 3: Summary statistics for quercetin and curcumin derivatives (measured as curcumin and quercetin after hydrolysis of derivatives) in urine; pharmacokinetics parameters**

| **URINE LEVELS** | | **Cmax (ng/mL)** | **%of administrated dose** | **Tmax (h)** |
|---|---|---|---|---|
| Total Curcumin (glucuronide + sulfate) | n | 12 | 12 | 12 |
| | Mean (SD) | 98.442 (52.183) | 0.084(0.027) | ∼8 |
| | CV% mean | 53.009 | 32.037 | |
| | Geo-mean | 85.645 | 0.080 | |
| | Median | 90.650 | 0.077 | |
| | [Min; Max] | [34.800 ; 184.700] | [0.04; 0.13] | |
| Total Quercetin (Aglycone + glucuronide + sulfate) | n | 12 | 12 | |
| | Mean (SD) | 8293.158 (6729.852) | 2.894 (1.377) | ∼0-4 |
| | CV% mean | 81.149 | 47.576 | |
| | Geo-mean | 6233.578 | 2.583 | |
| | Median | 5823.450 | 2.547 | |
| | [Min; Max] | [2096.2 ; 22058.4] | [1.31 ; 5.04] | |

The inventors then evaluated the efficacy of the composition of the invention (preferably the composition of Example 5) for the treatment of pollen allergic rhinitis in an environmental exposure chamber.

The inventors selected 24 patients suffering from seasonal allergic rhinitis and randomized them (50:50) in a placebo and in a treated group. The treated group received 2 capsules (one capsule: curcumin, 42 mg; Quercetin, 65 mg; citric acid 3.5 mg; and polysorbate 80 as emulsifier) of the invention, for 14 days, while the placebo group received 2 (visually) identical capsules with no curcumin and no quercetin.

The study was double-blind, randomized, placebo-controlled, two-arm cross-over.

The patients were selected following inclusion criteria such as age (18-65 years old), with a clinical history of Seasonal Allergic Rhinitis (SAR) with seasonal onset and offset of nasal allergy symptoms during each of the last two ragweed allergy seasons, and a positive skin prick test to ragweed allergen within 12 months prior to the first visit.

Patients were randomized to one of the treatment groups and be given instructions on how and when to take their medication. Patients then underwent a 14 days wash-out period and took after their daily dose at home for 15 days. Following the 15 days of treatment, subjects returned to the clinic for visit, and then entered the Environmental Exposure Chamber (EEC) and were exposed to pollen for 3 hours. Once in the chamber, the patients evaluated their nasal and ocular symptoms every 30 minutes for the three hours session. After a 14 days wash-out, patients then took the other medication (placebo or treatment) for 15 days. Following this period, subject returned to the clinic for an exposure similar to the precedent.

Overall, the inventors noticed a reduction in the Total Nasal Symptom Score (TNSS) in patients treated with the formulation of the invention containing curcumin, quercetin and an emulsifier, compared to placebo.

## Claims

1. A composition comprising in one capsule, an acid, a sufficient amount of curcumin, a sufficient amount of quercetin and a sufficient amount of an emulsifier, wherein the said sufficient amount of curcumin is comprised between 2 mg and 200 mg, the said sufficient amount of quercetin is comprised between 10 mg and 200 mg and the said sufficient amount of the emulsifier is comprised between 200 mg and 700 mg, the acid is comprised between 1 mg and 5 mg, and wherein the emulsifier has a viscosity at 25°C comprised between 0.100 Pa.s and 0.800 Pa.s for use in the treatment and/or the prevention of chronic rhinitis and/or allergic rhinitis.

2. The composition of claim 1 further encapsulated in gelatine capsules.

3. The composition according to the claim 1 or 2, wherein the acid is an organic acid selected from the group consisting of citric acid (E330), malic acid (E296), acetic acid (E260), tartaric acid (E334), lactic acid (E270), alginic acid (E400) and a mixture thereof.

4. The composition according to any of the claims 1 to 3, wherein the sufficient amount of the curcurmin is comprised between 10 mg and 100 mg and wherein the sufficient amount of the quercetin is comprised between 20 mg and 100 mg.

5. The composition according to any of the preceding claims, wherein the sufficient amount of curcumin is comprised between 5 mg and 50 mg and wherein the sufficient amount of quercetin is comprised between 20 mg and 100 mg.

6. The composition according to any of the preceding claims, further comprising a dryer.

7. The composition according to the claim 6, wherein the dryer is selected from the group consisting of cellulose, cellulose derivatives and SiO₂.

8. The composition according to claim 7, wherein the dryer is SiO₂ having a mean particle size comprised between 5 µm and 100 µm.

9. The composition according to any of the preceding claims which further comprises a sufficient amount of an essential oil, wherein said sufficient amount is comprised between 3% (w:w) to 30 % (w:w) of essential oil.

10. The composition according to claim 9 wherein the essential oil is selected from the group consisting of Agar oil, Ajwain oil, Anise oil, Balsam oil, Parsley oil, Bergamot oil, Cardamom seed oil, Carrot seed oil, Chamomile oil, Calamus oil, Cinnamon oil, Citronella oil, Fennel seed oil, Fenugreek oil, Frankincense oil, Galangal oil, Geranium oil, Ginger oil, Grapefruit oil, Henna oil, Juniper berry oil, Lavender oil, Lemongrass oil, Melissa oil, Mint oil, Myrrh oil, Orange oil, Oregano oil, Orris oil, Peppermint oil, Pine oil, Rosehip oil, Sage oil, Schisandra oil, Spikenard, Tarrangon oil, Tea tree oil, Thyme oil, Turmeric oil, Valerian oil, Yarrow oil and Zedoary oil.

11. The composition according to claim 9 wherein the essential oil is Turmeric oil.

## Patentansprüche

1. Eine Zusammensetzung, die in einer Kapsel eine Säure, eine ausreichende Menge an Kurkumin, eine ausreichende Menge an Quercetin und eine ausreichende Menge eines Emulgators umfasst, wobei die besagte ausreichende Menge an Kurkumin zwischen 2 mg und 200 mg beträgt, die ausreichende Menge an Quercetin zwischen 10 mg und 200 mg beträgt und die ausreichende Menge des Emulgators zwischen 200 mg und 700 mg beträgt, die Säure zwischen 1 mg und 5 mg beträgt, und wobei der Emulgator eine Viskosität bei 25 °C zwischen 0,100 Pa.s und 0,800 Pa.s aufweist, zur Verwendung bei der Behandlung und/oder Prävention von chronischer Rhinitis und/oder allergischer Rhinitis.

2. Die Zusammensetzung nach Einspruch 1, die ferner in Gelatinekapseln verkapselt ist.

3. Die Zusammensetzung nach Anspruch 1 oder 2, wobei die Säure eine organische Säure ist, die aus der Gruppe ausgewählt ist, bestehend aus Zitronensäure (E330), Apfelsäure (E296), Essigsäure (E260), Weinsäure (E334), Milchsäure (E270), Alginsäure (E400) und einer Mischung davon.

4. Die Zusammensetzung nach einem der Ansprüche 1 bis 3, wobei die ausreichende Menge des Kurkumins zwischen 10 mg und 100 mg beträgt und wobei die ausreichende Menge des Quercetins zwischen 20 mg und 100 mg beträgt.

5. Die Zusammensetzung nach einem der vorhergehenden Ansprüche, wobei die ausreichende Menge an Kurkumin zwischen 5 mg und 50 mg beträgt und wobei die ausreichende Menge an Quercetin zwischen 20 mg und 100 mg beträgt.

6. Die Zusammensetzung nach einem der vorhergehenden Ansprüche, die ferner ein Trocknungsmittel umfasst.

7. Die Zusammensetzung nach Anspruch 6, wobei das Trocknungsmittel aus der Gruppe ausgewählt ist, bestehend aus Zellulose, Zellulosederivaten und SiO₂.

8. Die Zusammensetzung nach Anspruch 7, wobei das Trocknungsmittel SiO₂ mit einer mittleren Partikelgröße zwischen 5 µm und 100 µm ist.

9. Die Zusammensetzung nach einem der vorhergehenden Ansprüche, die ferner eine ausreichende Menge eines essentiellen Öls umfasst, wobei die besagte ausreichende Menge zwischen 3% (Gew./Gew.) und 30% (Gew./Gew.) essentielles Öl umfasst.

10. Die Zusammensetzung nach Anspruch 9, wobei das essentielle Öl aus der Gruppe ausgewählt ist, bestehend aus Agaröl, Ajowanöl, Anisöl, Balsamöl, Petersilienöl, Bergamottöl, Kardamomsamenöl, Karottensamenöl, Kamillenöl, Kalmusöl, Zimtöl, Zitronellöl, Fenchelsamenöl, Bockshornkleeöl, Weihrauchpflanzenöl, Galgantöl, Geranienöl, Ingweröl, Grapefruitöl, Hennaöl, Wacholderbeerenöl, Lavendelöl, Zitronengrasöl, Melissenöl, Minzöl, Myrrhenöl, Orangenöl, Oreganoöl, Schwertlilienöl, Pfefferminzöl, Pinienöl, Hagebuttenöl, Salbeiöl, Spaltkörbchenöl, Narde, Estragonöl, Teebaumöl, Thymianöl, Gelbwurzelöl, Baldrianöl, Schafgarbenöl und Zitweröl.

11. Die Zusammensetzung nach Anspruch 9, wobei das essentielle Öl Gelbwurzelöl ist.

## Revendications

1. Composition comprenant dans une capsule, un acide, une quantité suffisante de curcumine, une quantité suffisante de quercétine et une quantité suffisante d'un émulsifiant, dans laquelle ladite quantité suffisante de curcumine est comprise entre 2 mg et 200 mg, ladite quantité suffisante de quercétine est comprise entre 10 mg et 200 mg et ladite quantité suffisante de l'émulsifiant est comprise entre 200 mg et 700 mg, l'acide est compris entre 1 mg et 5 mg, et dans laquelle l'émulsifiant a une viscosité à 25 °C comprise entre 0,100 Pa.s et 0,800 Pa.s pour utilisation dans le traitement et/ou la prévention de la rhinite chronique et/ou la rhinite allergique.

2. Composition de la revendication 1 qui est en outre encapsulée dans des capsules de gélatine.

3. Composition selon la revendication 1 ou 2, dans laquelle l'acide est un acide organique choisi dans le groupe constitué de l'acide citrique (E330), l'acide malique (E296), l'acide acétique (E260), l'acide tartrique (E334), l'acide lactique (E270), l'acide alginique (E400) et un mélange de ceux-ci.

4. Composition selon l'une quelconque des revendications 1 à 3, dans laquelle la quantité suffisante de la curcumine est comprise entre 10 mg et 100 mg et dans laquelle la quantité suffisante de la quercétine est comprise entre 20 mg et 100 mg.

5. Composition selon l'une quelconque des revendications précédentes, dans laquelle la quantité suffisante de curcumine est comprise entre 5 mg et 50 mg et dans laquelle la quantité suffisante de quercétine est comprise entre 20 mg et 100 mg.

6. Composition selon l'une quelconque des revendications précédentes, comprenant en outre un déshydratant.

7. Composition selon la revendication 6, dans laquelle le déshydratant est choisi dans le groupe constitué de la cellulose, de dérivés de cellulose et de SiO₂.

8. Composition selon la revendication 7, dans laquelle le déshydratant est SiO₂ ayant une taille de particule moyenne comprise entre 5 µm et 100 µm.

9. Composition selon l'une quelconque des revendications précédentes qui comprend en outre une quantité suffisante d'une huile essentielle, dans laquelle ladite quantité suffisante est comprise entre 3 % (m:m) à 30 % (m:m) d'huile essentielle.

10. Composition selon la revendication 9 dans laquelle l'huile essentielle est choisie dans le groupe constitué de l'huile d'agar, l'huile d'ajowan, l'huile d'anis, l'huile de sapin baumier, l'huile de persil, l'huile de bergamote, l'huile de graines de cardamome, l'huile de graines de carotte, l'huile de camomille, l'huile de calamus, l'huile de cannelle, l'huile de citronnelle, l'huile de graines de fenouil, l'huile de fenugrec, l'huile d'encens, l'huile de galanga, l'huile de géranium, l'huile de gingembre, l'huile de pamplemousse, l'huile de henné, l'huile de baies de genièvre, l'huile de lavande, l'huile de citronnelle, l'huile de mélisse, l'huile de menthe, l'huile de myrrhe, l'huile d'orange, l'huile d'origan, l'huile d'iris, l'huile de menthe poivrée, l'huile de pin, l'huile de rose musquée, l'huile de sauge, l'huile de schisandra, l'huile de nard, l'huile d'estragon, l'huile d'arbre à thé, l'huile de thym, l'huile de curcuma, l'huile de valériane, l'huile d'achillée millefeuille et l'huile de zédoaire.

11. Composition selon la revendication 9 dans laquelle l'huile essentielle est l'huile de curcuma.
